# EUROPEAN PATENT APPLICATION

(11) **EP 1 002 537 A1**
(43) Date of publication of application: **24.05.2000**
(21) Application number: 98402714.4
(22) Date of filing: 30.10.1998
(51) Int. Cl.: A61K 31/53

(54) **Use of melarsoprol for the manufacture of a medicament for treating B-cell lymphoproliferative diseases, such as multiple myeloma**

(71) Applicant: ASSISTANCE PUBLIQUE, HOPITAUX DE PARIS, 75004 Paris (FR); UNIVERSITE PARIS 7 - Denis DIDEROT, F-75005 Paris (FR)
(72) Inventor: Fermand, Jean-Paul, 93340 Le Raincy (FR); Rousselot, Philippe, 75012 Paris (FR)
(74) Representative: Le Forestier, Eric

(57) **Abstract**

The present invention concerns new uses of melarsoprol for the preparation of pharmaceutical compositions for treating B-cell lymphoproliferative disorders, preferentially Multiple Myeloma.

## Description

### Field of the invention

The present invention is directed to the new use of melarsoprol (Arsobal®) in the treatment of B-cell lymphoproliferative disorders, preferentially Multiple Myeloma.

### Background of the invention

Melarsoprol (Arsobal®) is the trivial name designating 2-p-[(4,6-diamino-s-triazin-2-yl)amino]phenyl-4-hydroxymethyl-1,3,2-dithiarsoline. It is a well-known arsenic-derivative of formula :

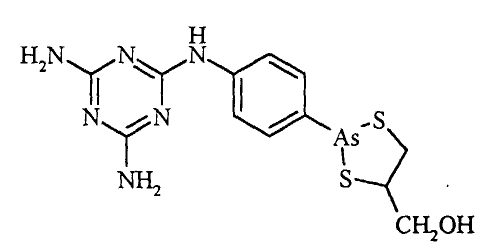

Melarsoprol has been one of the reference drug used since the 1950s, for treatment of late stage Trypanosomia and sleeping sickness with involvement of the central nervous system. Melarsoprol is the drug of choice for patients with central nervous system (CNS) involvement, because it crosses the blood-brain barrier.

The 2,3-dimercaptopropanol adduct of melarsen oxide is formed using the metal-chelating drug dimercaprol. The drug is thought to interact with dithiol trypanothione, novel to trypanosomatids but analogous to mammalian glutathione, leading to the inhibition of trypanothione reductase central to the regulation of the thiol/disulfide redox balance in the parasite absent from the host.

According to the recommendations of the World Health Organisation, the drug is administered according to complicated dosage and schedules during a total of three to four weeks. Basically, patients receive eventually escalating doses of Melarsoprol intravenous for 3 days, repeated for several weeks. The complicated dosage schedules are based on clinical experience only as the pharmacokinetics and metabolism of Melarsoprol are unknown. Side-effects are common, the most serious being encephalopathy, which occurs in 2 to 10% of the patients (Journal of chromatography, 690, 1997, 243-251). Other side-effects are albuminuria and abdominal pain. Also nausea, vomiting, diarrhea, dermatitis and allergy may occur.

However, there is no knowledge of relationships between drug concentrations and side effect. The Lancet (1989, 1246-1249) reported side-effects associated with

melarsoprol therapy comprising encephalopathy, polyneuropathy, cutaneous reaction, infections such as pneumonia, meningitis, tropical myositis, suppurative adenitis, bacterial dysentery and perforated otitis media.

In order to prevent the encephalopathy occurring during melarsoprol treatment, corticosteroids have been used before melarsoprol treatment to 〈〈 prepare 〉〉 the patient, during treatment and even after treatment (Bertrand et al, Bull.Soc.Pathol.Exot.Filiales 1968(61), 617-625). For instance, Pepin et al,The Lancet 1989, 1246-1249 had shown that the reduction in the frequency of reactive encephalopathy in patients who were given prednisolone led to a decrease in encephalopathy associated death during melarsoprol treatment; the efficacy of prednisolone in preventing this complication is a strong argument in favour of an autoimmune pathogenesis of melarsoprol induced encephalopathy because steroids are unlikely to decrease direct arsenical toxicity.

Inorganic arsenic-containing compounds were used in cancer treatments in the early 1900s.

For instance, potassium arsenite (Fowler's solution) was used to control elevated leukocyte counts in chronic myelogenous leukemia (Forkner et al, JAMA 97:305,1931).

The only arsenical currently formulated for human use in Western countries is Melarsoprol.

Consequently, there is a need to demonstrate possible anti-cancer activity of Melarsoprol.

Insofar, most of the recent investigations on melarsoprol in the oncology area have been carried out to demonstrate its anti-leukemia properties.

Leukemia are acute or chronic neoplastic disorders marked by an abnormal and often severe proliferation of leucocytes and cells of their genealogical lines.

In this context, the activity of both Melarsoprol and Arsenic trioxide have been widely compared.

In a study by R.Rivi et al, Melarsoprol shows *in vitro* growth suppressive activity via programmed cell death on chronic and acute myeloid and lymphoid leukemia derived cell lines. See Blood, 88(10), 1996, 68a.

A.König et al. "Comparative activity of Melarsoprol and Arsenic trioxide in chronic B-cell leukemia lines", Blood, 90(2), 1997, 562-570, reported that Melarsoprol (10-7 to 10-9 mol/l) exhibits *in vitro* activity in some specific lymphocytic leukemia cell lines (JVM-2, I83CLL, and WSU-CLL): the inhibition of growth in all three cell-lines was achieved by promoting programmed cell death. By contrast, no effect was obtained with arsenic trioxide.

Other tests conducted by Z.G.Wang et al , Blood, 90(10), 1997, 327a shown that arsenic trioxide and melarsoprol induce programmed cell death in myeloid leukemia cell lines.

Moreover, it has been reported by Z.Xie that Melarsoprol and arsenic trioxide could overcome multidrug resistance (see Blood, 90(10), 1997, 495a).

Recent reports from several institutions suggest that melarsoprol induces apoptosis similar to that of AS₂O₃. Researchers at Memorial Sloan-Kettering (Konig et al., Blood; 90(2), 1997) reported that melarsoprol inhibits the growth of lymphoid leukemia cells by promoting programmed cell death. In addition, melarsoprol together with all-trans-retinoic acid (ATRA) has been reported to inhibit both breast and prostate tumor cells *in vitro* as well as *in vivo* (Falagana, et al., Proc AACR, 39, 1998). This particular combination appears to induce the downregulation of bcl-2 with the concomitant upregulation of p21.

However, all of these encouraging *in vitro* results showing the possible activity of Melarsoprol on leukemia were only obtained on cell lines. They must then be confirmed *in vivo* and on patients studies.

During clinical studies in leukemia, S.Soignet et al (see Proceedings of ASCO, 1997, 4a) observed no major anti-leukemic activity although they had found that melarsoprol exhibited broad *in vitro* activity.

In this study, patients received escalating doses of melarsoprol intravenous(IV) daily for 3 days, repeated weekly for 3 weeks. Doses were 1mg/kg on day 1, 2mg/kg on day 2, and 3,6mg/kg on day 3 and thereafter (maximum dose = 200mg).

According to the same dose regimens, no major antileukemic effects in AML (accute myeloid leukemia) and CML (chronic myeloid leukemia) were observed; one patient with CLL (chronic lymphoid leukemia) had substantial regression of both peripheral lymphadenopathy and splenomegaly).

However, it has been reported that this dose caused excessive CNS (central nervous system) toxicity; according to the authors, 〈〈 further studies are needed to determine the optimal dose, schedule and potential efficacy〉〉. see S.Soignet et al, Blood 90(10), 1997, 507a.

The above mentioned administration protocol in leukemia treatment is the same one as that one recommended in the treatment of Trypanosomiasis and severe side-effects occurred.

Therefore this 〈〈 sequential 〉〉 administration schedule is unsatisfactory and a schedule of administration of melarsoprol efficient for treating neoplastic diseases and/or with minimized side-effects is still needed.

On another hand, it has been investigated whether melarsoprol could also exhibit activity on B-cell lymphoproliferative disorders, distinct from leukemia, such as multiple myeloma.

Multiple myeloma and related disorders comprise a spectrum of diseases that are characterized by autonomous proliferation of differentiated plasma cells, plasmocytes, whose physiologic function is to secrete immunoglobulins. Multiple myeloma is the most common primary neoplasm of bone. The bone marrow is usually extensively involved by myeloma and patients respond poorly to therapy (Atlas of diagnostic oncology, A.T.Skarin, Mosby-Wolfe).

J.P. Fermand et al, Blood, 90(10), 1997, 325a have shown that Arsenic trioxyde and Melarsoprol induce myeloma cell apoptosis *in vitro* with a preferential effect on tumoral cells in patients' bone marrow.

However, this *in vitro* preliminary study must be confirmed on patients ; the need of such a confirmation is more acute, considering the variabilities of results between *in vitro* and clinical results, especially as emphasized by the above-mentioned prior art related to leukemia.

Moreover, no satisfactory administration scheme has been insofar established.

Moreover, the mechanism of action of melarsoprol is not yet fully understood.

Currently, the potential mechanism of action of melarsoprol as a chemotoxic agent against tumor cells is unknown. Published observations suggest that treatment of cell lines with Arsobal leads to the induction of apoptosis correlated with a down-regulation of bcl-2. However, this does not establish a causal relationship between the action of the drug and the observed result because other agents (e.g., the taxanes) elicit similar indirect effects.

Perhaps more intriguing is Melarsoprol's effect on mammalian glutathione reductase (GR), known to regulate the levels of intracellular glutathione in response to oxidative stress. Published data suggests that chemoresistance to cisplatin and doxorubicin in some tumors may be related to elevation of the GR detoxification enzyme. Since melarsoprol also inhibits GR, although to a lessor degree than trypansomal form of the reductase, Arsobal might serve to increase cellular sensitivity to oxidative stress. As such, it could serve as an agent to treat chemoresistant tumors or enhance the efficacy of radiation therapy.

There is significant unmet clinical need for B-cell lymphoproliferative disorders, particularly multiple myeloma.

The therapeutic populations for multiple myeloma are estimated at approximately 25,000 in the US and 3200 in France. Estimates for the leading nation markets total 51,000 patients.

There is therefore an unmet need to develop a schedule of administration of melarsoprol for treating for B-cell lymphoproliferative disorders, particularly multiple myeloma, and still preferentially causing less side-effects.

It is highly desirable that the treatment with melarsoprol should provide the patient sufficient antineoplastic effect, while preferentially not exceeding dose-limiting toxicities

It is also desirable to minimize pre- and co-medication since this increases patient discomfort and increases the expense and duration of treatment.

Even if treatment duration can not be shortened, it is also desirable to a schedule different from the well-known dosage which induced a variety of adverse side-effects including encephalopathy, polyneuropathy...

Therefore, it could be desirable to optimize melarsoprol dosages to reduce the toxic side effects of the treatment.

It is also highly desirable to decrease the period required to administer melarsoprol to patients to minimize patient discomfort and expense.

Another need is the new use of melarsoprol for the preparation of pharmaceutical composition for treating B-cell related disorders, preferentially multiple myeloma, and the administration schedule of the medicaments containing melarsoprol for treating B-cell related disorders, preferentially multiple myeloma.

### Summary of the invention

This is one object of the present invention, to provide the new use of melarsoprol for the preparation of pharmaceutical compositions for treating B-cell lymphoproliferative disorders, particularly multiple myeloma.

This is another object of the present invention to provide new use of melarsoprol for the preparation of medicaments for treating B-cell lymphoproliferative disorders for administration according to a dosage schedule suitable for treating B-cell lymphoproliferative disorders, preferentially while minimizing the side effects induced by the administration of melarsoprol.

A preferred embodiment is to provide new use of melarsoprol for the preparation of medicaments for treating multiple myeloma for administration according to a dosage schedule suitable for treating multiple myeloma, preferentially while minimizing the side effects induced by the administration of melarsoprol.

Another preferred object of the invention is to provide with a method of treatment of B-cell lymphoproliferative disorders, preferentially multiple myeloma with melarsoprol.

A more preferred object of the invention is to provide with an appropriate administration protocol for treating multiple myeloma, which would allow the decrease of side-effects such as neurotoxicity.

### Description of the invention

This is one object of the present invention, to provide the new use of melarsoprol for the preparation of pharmaceutical compositions for treating B-cell lymphoproliferative disorders, particularly multiple myeloma.

This is another object of the present invention to provide new use of melarsoprol for the preparation of medicaments for treating B-cell lymphoproliferative disorders for administration according to a dosage schedule suitable for treating B-cell lymphoproliferative disorders, preferentially while minimizing the side effects induced by the administration of melarsoprol.

A preferred embodiment is to provide new use of melarsoprol for the preparation of medicaments for treating multiple myeloma for administration according to a dosage schedule suitable for treating multiple myeloma, preferentially while minimizing the side effects induced by the administration of melarsoprol.

In another embodiment, the present invention is concerned with a process of preparation of a medicament for treating B-cell lymphoproliferative disorders, preferentially multiple myeloma characterized in that melarsoprol is the main active ingredient of the said medicament.

In another embodiment, the present invention is concerned with a solution containing melarsoprol at concentration of 36mg/ml for treating B-cell lymphoproliferative disorders , preferentially multiple myeloma.

The hereabove and hereafter so-called B-cell lymphoproliferative disorders comprise multiple myeloma, and other B-cell lymphoproliferative disease such as chronic lymphoid leukemia (CLL), B-prolymphocytic leukemia (PLL), Waldenstrom's macroglobulinemia and B-non-Hodgkin lymphomas.

Among these hereabove and hereafter mentioned B-cell lymphoproliferative disorders, multiple myeloma is of particular interest.

### 1 - New use of melarsoprol for the preparation of pharmaceutical compositions for treating B-cell lymphoproliferative disorders, preferentially multiple myeloma.

According to the present invention, melarsoprol reduces the growth and survival of myeloma cells by triggering programmed cell death. The apoptotic effect of the arsenical compound was observed in plasma cell lines and in malignant plasma cells freshly isolated from patients with multiple myeloma and the melarsoprol-induced apoptosis was not rescued by IL-6.

This supports the clinical efficacy of the melarsoprol in patients with multiple myeloma. Indeed, melarsoprol is active at concentrations ranging from 10⁻⁶ to 10⁻⁸ mol/L, which correspond to the *in vivo* pharmacological concentrations of the drugs.

In contrast, at pharmacological concentrations of melarsoprol, reduction in survival and growth of other tested cell types, either lymphoid, myeloid or epithelial was less marked than in promyelocytic and plasma cells.

The selective effect on myeloma cells of melarsoprol was illustrated by experiments performed in bone marrow mononuclear cells freshly harvested from patients with mutiple myeloma, which showed a marked decrease in myeloma cell viability with, conversely, only minimal changes in myeloid cells. These were mainly a decrease in the number of CD38^{low}, CD15^{low} positive cells which include monocytes and myeloid progenitors.

### 2 - New use of melarsoprol for the preparation of medicaments to be administered according to an new dosage schedule, for treating B-cell lymphoproliferative disorders

Despite the poor knowledge of the mechanism of action of melarsoprol, it has been surprisingly discovered how to use melarsoprol for chemotoxic activity in B-cell lymphoproliferative disorders, preferentially multiple myeloma.

Despite the conventional practice that it is necessary to administrate melarsoprol IV sequentially (daily for 3 days, repeated weekly), it is one object of the present invention that melarsoprol can be used for the preparation of medicaments for treating B-cell lymphoproliferative disorders, preferentially multiple myeloma for administration via 〈〈 continuous 〉〉 dosage schedule.

According to use of the present invention, the 〈〈 continuous 〉〉 schedule mentioned hereabove and hereafter means at least 5 consecutive days a week, preferentially daily.

According to the use of the present invention, melarsoprol is preferentially admistered to doses which can depend on the schedule used, preferentially, the dose on day 1 is smaller than the doses administered the consecutive days after, and still preferentially, the doses do not escalade after day 2.

According to the use of the present invention, melarsoprol is preferentially administered at dosages such as its blood concentration is between 10⁻⁸ and 10⁻⁶ mol/l; still preferentially, at dosages of 1 mg/kg to about 3mg/kg.

According to the use of the present invention, melarsoprol is preferentially administered by intravenous route.

According to the use of the present invention :
in a preferred embodiment, the melarsoprol is administered according to this scheme to treat multiple myeloma;
in a still preferred embodiment , melarsoprol is administered IV daily at dosages of about 1mg/kg to about 3mg/kg;
in another preferred embodiment, melarsoprol is administered via intravenous injections, preferentially 3 intravenous injections per day.

In a still most preferred embodiment, melarsoprol is administered IV daily 1mg/kg day one, and then 2,2 mg/kg daily, preferentially via 3 intravenous injections.

According to the use of the present invention, melarsoprol is in form of a injectable solution, preferentially at concentration equal to 36mg/ml, and still preferentially in propylenglycol.

Of great significance is a surprising discovery that the continuous schedule causes less side-effects, commonly associated to the melarsoprol, such as encephalopathy, CNS toxicity.

Following the preferred schedules of the present invention provides objective response rates for patients suffering from B-cell lymphoproliferative disorders, preferentially such as multiple myeloma.

The surprising effect that melarsoprol could be administered via a continuous schedule, preferentially daily, means that it will now be possible to use it safely to treat B-cell lymphoproliferative disorders, such as multiple myeloma.

While experimental data is provided herein for the successful treatment of multiple myeloma with melarsoprol using the present products and treatment protocol, it is to be understood that the products and treatment protocol can be used for the treatment of other forms of neoplastic diseases of the hematopoietic and lymphoid tissues, such as :
systemic diseases comprising acute leukemias, chronic lymphoid leukemias and other lymphoproliferative diseases, chronic myeloid leukemia and other myeloproliferative diseases, chronic monocytoid leukemia and histiocytoid diseases, as well as
tumours comprising lymphosarcomas, mycosis fungoides, plasmacytoma, reticulosarcoma, unclassified lymphomas, hodgkin's disease, and
the other diseases according to the international histological classification of tumours established by the WHO (classification histologique et cytologique des maladies néoplasiques des tissus hématopoïétiques et lymphoïdes, Organisation mondiale de la santé, 1977, Genève).

B-cell lymphoproliferative disorders are preferred according to the present invention.

A bi-central, open, non-comparative study of melarsoprol in patients suffering from secreting multiple myeloma was carried out.

Patients with secreting multiple myeloma included in the study had been pretreated with and were unresponsive to a minimum of 3 VAD (vincristine-adriamycine-dexamethasone) or VAMP (vincristine-adriamycine-methylprednisolone) chemotherapy regimens.

These regimens were administered else during a relapse following an intensive treatment with self-transplanting with hematopoïetic precursors, or else after many regimens with at least one chemotherapy comprising alkylating agents.

VAD- or VAMP- drug refractory diseases do currently have very few therapeutic possibilities and life expectancy of patients suffering from these diseases is very limited, These diseases comprising myeloma require therefore new treatments efficient wherein all known treatments failed.

The response obtained with the treatment with melarsoprol according to the invention is truly expected since responses to drug refractory myeloma are extremely uncommon.

Patients included in the study are required to have received no chemotherapy (except corticoids) for at least 4 weeks or to have recovered from toxicity of the last chemotherapeuties administered.

Patients are treated daily with co-administration of anti-convulsive and B1 vitamin for 56 days if allowed by toxicity.

Non-inclusion criteria concern patients who are suffering from non secreting myeloma, or are non administered with at least 2 sequences of different chemiotherapies, or are administered neither with VAD, nor with VAMP, or pregnant or breast-feeding woman, or patients suffering from one of the following contra-indications :
unsufficient renal, hepatic, cardiac, respiratory functions, diarrhea, infection, previous administration with all-trans retinoic acid or one of its derivatives, α2-interferon, cytokine, hematopoietic growth factor.

Melarsoprol (Arsobal®) was supplied by RHONE-POULENC RORER as concentrated sterile solution for IV administration.

Each 180 mg ampoule contains 36mg/ml melarsoprol diluted in propylenglycol.

Melarsoprol must be used without further dilution.

At the first day, the IV solution of melarsoprol (1mg/kg) is administered in 3 intravenous injections.

The IV solution of melarsoprol (2mg/kg) is then administered daily and for a time period not longer than 56 days in the same manner.

The melarsoprol treatment can be associated with the following comedication :
- B1 vitamin : at least 250 mg/day IV
- Rivotril ® : 1 mg/day PO or IV or another anticonvulsive agent.

### Efficacy and safety

Responses are evaluated according to the criteria commonly used for the disease :
- complete remission : less than 5% of medullary plasmocytes with normal cytologic aspect and disappearance of the abnormal monoclonal component, detected by immuno-electrophoresis and immunofixation in serum and on a 100 fold concentrated urine sample.
- minime residual disease : less than 5% of medullary plasmocytes and at least 90% decrease of the abnormal serum amonoclonal component rate.
- partial response : at least 50% decrease of the abnormal serum amonoclonal component rate and/or at least 50% decrease of the Bence-Jones urinary proteinuria.
- minor response : at least 25% decrease of the abnormal serum amonoclonal component rate and/or at least 50% decrease of the Bence-Jones urinary proteinuria.
- failure : less than 25% and 50% decrease of the respective serum and urinary abnormal monoclonal component rates, or, else, progression of the disease. Progression is defined by one of the following criteria :
   - at least 25% increase of the serum monoclonal immunoglobuline rate
   - at least 50% increase of the Bence-Jones proteinuria rate
   - hypercalcemy
   - extension of the pre-existing plasmocytes
   - appearance of new tumoral injuries
The response is evaluated :
- by the end of the 56 day protocol,
- or by possible arrest of the protocol, if toxic event occurs,
- or by progression of the disease occurring during the second treatment month,
- or by the third month after the end of the treatment.

Efficiency of melarsoprol for treating multiple myeloma and reduced side-effects according to the above-mentioned dosage schedule are verified.

### Examples

### I. Reprint from Rousselot et al "Arsenic trioxyde and melarsoprol induce apoptosis in plasma cell lines and in plasma cells from myeloma patients", submitted for publication.

### Reagents.

Melarsoprol (supplied as Arsobal [36 mg/ml] by Rhone Poulenc Rorer. Paris) was stored at room temperature and diluted to working concentration in propylene glycol Human recombinant interleukine-6 (IL-6) and α - interferon (α - INF) were obtained from Diaclone (Besançon. France) and Hoffmann La Roche (Bale. Switzerland), respectively.

### Cell lines

The human myeloma cell lines were purchased from DMS (Braunschweig, Germany) (NCI H929 and LP1) and from American Type Culture Collections (Rockville, MD) (OPM2 and U266). NCI, OPM2, U266 (and all studied non plasmacytic cell lines) were cultured in RPMI 1640 supplemented with 1% penicillin/streptomycin, 1 mmol/L L-glutamine and 10% heat-inactivated fetal calf serum at 37°C, 5% CO₂ in air. LP1 cells were cultured in similar conditions, in Iscove's medium.

### Patent samples

Eleven patients with multiple myeloma (MM) or plasma cell leukemia (PCL) were studied after informed consent. Mononuclear cells were separated by Ficoll-Hypaque centrifugation from peripheral blood of 4 patients with PCL (including 2 with a primary PCL), from bone marrow (BM) of 6 patients with MM (including 4 at diagnosis) and from the pleural effusion of a newly diagnosed myeloma patient. Plasma cell-enriched preparations were obtained by centrifugation of mononuclear cells on a Percoll gradient, as previously described (Iwato K, et al, Blood 72:562, 1988). Plasma cell percentages were determined after intracytoplasmic staining using fluorescein-conjugated antibodies to human immunoglobulin (Ig) heavy and light chains (from Nordic, Tilburg, The Netherlands).Short-term cultures of blood and BM mononuclear cells and of plasma cell-enriched preparations were performed in RPMI 1640, in similar conditions as cell lines.

### Pokeweed mitogen (PWM)-induced differentiation of normal lymphocytes

Mononuclear blood cells of 3 normal donors were cultured in the presence of PWM (from Life Technology, Cergy Pontoise, diluted at 1/100) and melarsoprol (5 x 10⁻⁷ or 10⁻⁶ mol/L). Plasma cells were numerated at day 7 after immunofluorescence staining with fluorescein-conjugated antibodies directed against human Ig chains, IgM concentrations in supernatants were also measured at day 5, by ELISA, as previously described (Grillot-Courvalin C et al, Scand. J. Immunol. 23:679, 1986).

### Viability, cell morphology and cell proliferation

Cells were incubates with or without 10⁻⁵ to 10⁻⁸ mol/L melarsoprol up to 96 hours, without any adjustment after culture initiation. Cell number and viability were determined by trypan blue exclusion. Morphologic analysis was performed using Wright-Giema and DAPI (4'-6-diamidino-2-phenylindole) staining on cytocentrifuge preparations. For proliferation assays, aliquots of 2x10⁴ cells in triplicate were incubated in 96-well plates and 3H-thymidine (3H-TdR) (1 µCi) was added for the final 8 hours of the culture period. Cells were harvested onto glass filter and ³H-TdR incorporation was counted on a liquid scintillation counter.

### Flow cytometry assay

Direction of phosphatidylserine on the outer leaflet of apoptotic cells was performed using Annexin-V-FLUOS (Boehringer Mannheim, Germany) and propidium iodide (PI) according to the manufacturer's recommendations. Expression of cell surface CD38, CD138 (BB-4° and CD15 antigens was studied using corresponding fluorescein isothiocyanate (FITC) or phycierythrin conjugated antibodies that were purchased from Diaclone (Besançon, France) and Becton Dickinson (San Jose, Ca.). Flow cytometry was performed with a FACSean apparatus (Beckton Dickinson).

### TUNEL assay

Apoptosis was also assessed by deoxyuridine triphosphate [dUTP] labeling of DNA nicks with terminal deoxynucleotidyl transferase (TdT). The TUNEL (TdT-mediated dUTP nick-end labeling) assay was performed by use of the IN Situ Cell Death direction kit (Boehringer Mannheim, Germany). DNA strand breaks were identified on cytospin slides by labeling free 3'-OH termini with FITC-conjugated dUTP. Incorporated fluorescein was detected by anti-fluorescein antibody Fab fragments from sheep, conjugated with alkaline phosphatase. After substrate reaction, stained cells were analyzed under light microscope.

### Immunofluorescence study of PML

Indirect immunofluorescence staining of PML was performed to a previously described method (17) using rabbit polyclonal antibodies specific to the N-terminal region of PML (kindly provided by H. De The, The Saint Louis Hospital, Paris). In some cases, double intracytoplasmic stainings were performed rising rabbit antibodies to PML and pig fluorescein-conjugated anti-rabbit Ig antibodies (Dako, Danmark) followed by goat rhodamine-conjugated Fab'₂ antibodies directed against human Ig (Nordic, Tilburg, The Netherlands).

### in vitro results

This activity of melarsoprol has been investigated on multiple myeloma cell lines.

Studies have been conducted on 4 plasma cell lines (NCI, U266, OPM2, LP1) :

The cells have been cultivated in the presence of melarsoprol at different concentrations.

### Melarsoprol causes a dose- and time-dependent inhibition of growth in human myeloma cell lines.

The growth of NCI, U266, OPM2 and LP1 plasma cell lines in medium alone or in the presence of melarsoprol (10⁻⁵ to 10⁻⁸ mol/L) was assessed by cell count and thymidine incorporation. Treatment of NCI cells with different concentrations of melarsoprol during 72 hours induced a marked does-dependent decrease in cell number and thymidine incorporation with a 50% growth inhibitory concentration (IC50) of 3.10⁻⁷ mol/L. IC50 was also in the order of 10⁻⁷ mol/L for OPM2 cells and was about 10⁻⁶ mol/L for U266 and LP1 cells.

The arsenical compound also led to a marked time-dependent inhibition of growth in NCI and OPM2 cells, and to a significant but less important inhibition of growth in U266 and LPI cells.

### Comparative example :

In addition to plasma cell, other hematopoietic cell types were also studied. The inhibition of growth was marginal in T, myeloid and epithelial cell lines.

### Melarsoprol reduces the viability and induces apoptasis in myeloma cell lines.

Treatment of plasma cell lines with melarsoprol led to a dose- and time dependent inhibition of cell survival, as determined using trypan blue exclusion. After 48 hours, in the presence of 10-6 mol/L melarsoprol , percentage of trypan blue negative cells were 15 ± 7.2, 56 ± 11, 62 ± 5 8, and 81 ± 6.8% in NCI, OPM2, U266 and LP1 cells, respectively. Cell death was mainly due to apoptosis, as shown by the characteristic morphological changes in nuclei as well as by the TUNEL method. After 24 hours of culture with 10⁻⁶ mol/L melarsoprol, a significant percentage of cells exhibiting DNA strand-breaks (between 10 and 70% according to the cell line, as compared to 3% or less in control cultures) were detected by in situ TdT assay. DNA fragmentation was confirmed by agarose gel electrophoresis of genomic DNA extracted from NCI cells treated with 10⁻⁶ mol/L melarsoprol which showed typical DNA ladders corresponding to internucleosomal cleavage.

FACS analysis of NCI and U266 cells after staining with fluorescein-labelled annexin-V and propidium iodide was performed to detect the translocation of phosphatidylserine from the inner part of plasma membrane to the outer layer which accurs in the early stages of apoptosis. Before staining, cells were cultivated with medium alone) melarsoprol (10⁻⁶ mol/L) or dexamethasone (Dex, 10⁻⁶ mol/L) over a time period of 48 hours. Treatment of NCI cells with melarsoprol rapidly induced apoptosis as demonstrated by an increasing percentage of annexin-positive, PI-negative apoptotic cells. During the same time; the number of double positive necrotic cells increased and, at 18 hours, only very few double-negative living cells were detectable. Apoptosis was moderate in cells cultured with Dex and in control culture. Annexin-positive, PI-negative apoptotic U266 cells were also observed in the presence of melarsoprol (18% of residual cells after a 18-hour culture).

### Melarsoprol decreases viability and cell growth and induces apoptosis in fresh myeloma cell

Melarsoprol also inhibits the viability (trypan blue exclusion) and growth (3 H TdR uptake) of plasma-cell enriched preparations (containing more than 80% of plasma cells) from bone marrow (n=6), blood (n=4) and pleural effusion (n=1) of 11 myeloma patients. Exposure to melarsoprol for 48 hours induced a mean decline in cell viability to 52.5 ± 17.3% (40 to 77.5%, n=4) respectively, as compared with 10.5 ± 14.7% (0 to 27%, n=9) in cultures performed without the drug. In the three plasma-cell preparations that were cultured in the presence of different concentrations of the arsenical compound, a dose-dependent inhibition of cell growth was observed, with an IC50 in the order of 10⁻⁷ mol/L for melarsoprol. A significant percentage of apoptotic cells (up to 50% and 90% at day 2 in the presence of 10⁻⁶ mol/L melarsoprol, as compared with 5 to 19% in control cultures) was demonstrated by the TUNEL method in all studied samples (n=4).

### Preferential effect of melarsoprol on myeloma cells in patients' bone marrow

To comparatively assess the effects of melarsoprol on myeloid and plasma cells, bone marrow mononuclear cells obtained from patients with newly diagnosed multiple myeloma were cultured for 5 days in the presence of 10⁻⁶ mol/L melarsoprol. FACS analysis of bone marrow cells after double staining with fluorescein-labelled annexin V and phycoerythrin labelled CD38 or CD15 antibodies demonstrated that apoptotic cells were mainly myeloma cells. Indeed, in a representative study a bone marrow sample initiality containing 30% of plasma cells (CD38^{higt} +), 12% of the latter expressed annexin V after 2 days of culture with 10⁻⁶ mol/L melarsoprol, whereas less than 1% of myeloid cells (CD38^{low}, CD15 +) were positive for the apoptosis marker. At day 5, the plasma cell number had much more decreased than the myeloid cell number and viable cells (propidium iodide negative) contained 7.8 % of CD38^{higt} positive cells, as compared to 22.5 % in control culture, performed with medium alone. CD15+ myelo-monocytic cell percentages were similar in arsenic-exposed cultures and in controls, with a decrease in the CD15^{low}+ fraction.

### Interleukine-6 (IL-6) and α-interferon (α-IFN) did not influence the inhibitory effect of melarsoprol

Since IL-6 have been shown to prevent the apoptosis of myeloma cells exposed to serum starvation or Dex (Oncogene, 15, 837, 1997), we assayed its effect on the viability and growth of plasma cell lines and primary myeloma cells treated with melarsoprol. NCI and U266 cells and plasma cell enriched preparations from 5 patients were studied. Recombinant IL-6 alone (100 pg/ml) slightly enhanced the proliferation of all tested cell types. However, whether it was added simultaneously or before the arsenical compound (10⁻⁶ mol/L to 10⁻⁷ mol/L), IL-6 did not prevent either cell death or growth inhibition.

The effect of α-IFN was similarly studied. α-IFN alone (100 or 1000 UI/ml) had a dose-dependent anti-proliferative effect on cell lines as well as on fresh plasma cells. In most cases, the combination of α-IFN with melarsoprol had only minimal effect on the growth inhibition induced by the arsenical compound alone.

### Effect of melarsoprol on plasma cell differentiation of normal B cells

Mononuclear cells purified from peripheral blood of normal donors were incubated with pokeweed mitogen in the presence of two different concentrations (10⁻⁶ mol/L and 5 x10⁻⁷ mol/L) of the organic or of the inorganic arsenical. In all cases, melarsoprol, including at 10⁻⁶ mol/L, only slightly inhibited the plasma cell differentiation induced by the mitogen. In contrast, the number of plasma cells and the amount of secreted IgM were markedly reduced in cultures performed with PWM plus As₂O₃, as compared with PWM alone.

### II. In vivo results

The following studies of melarsoprol conducted in SCID mice transplanted with human myeloma cells confirm in vivo the in vitro effects of the arsenical compound.

SCID mice were irradiated before the intra-peritoneal (IP) injection of 3 to 5*10⁶ plasma cells purified from peripheral blood of patients with plasma cell leukemia. Engrafment was assessed by bi-monthly measurement of the human monoclonal immunoglobulin (HuMIg) concentration in mouse serum, using an ELISA. Treatment with melarsoprol (30µg/g 5 days a week IP) was started when a sustained growth of the tumor cells was demonstrated by a progressive increase in HuMIg amounts at 3 consecutive dosages.

Mice injected with the cells from 3 different patients developed the human tumor and were treated with melarsoprol during 30 to 40 days. In all evaluable experiment remissions have been achieved as marked by a dramatic decrease in the level of the HuMIg which even became undetectable in the serum of some animals.

There was no evidence of toxic lesions in any studied organ, especially in the liver. Arsenic amounts in mouse serum and tissues were in the expected 〈〈 pharmacological 〉〉 range.

## Claims

1. Use of melarsoprol for the preparation of pharmaceutical compositions for treating B-cell lymphoproliferative diseases

2. Use according to claim 1 wherein the B-cell lymphoproliferative disorder is multiple myeloma.

3. Use of melarsoprol for the preparation of medicaments for treating B-cell lymphoproliferative disorders for the administration according to a continuous dosage schedule.

4. Use according to claim 3 wherein the B-cell lymphoproliferative disorder is multiple myeloma.

5. Use of melarsoprol according to claim 3 to 4 wherein the schedule is daily.

6. Use of melarsoprol according to claim 3 to 5 wherein melarsoprol is admistered at constant doses after day 2.

7. Use of melarsoprol according to claim 3 to 6 wherein the side effects induced by the administration of melarsoprol are minimized.

8. Use according to anyone of the previous claims 3 to 7 wherein melarsoprol is administered IV.

9. Use according to anyone of the previous claims 3 to 8 wherein melarsoprol is administered at dosages of about 1 mg/kg to about 3 mg/kg.

10. Use according to anyone of the previous claims 3 to 9, wherein melarsoprol is administered IV daily 1mg/kg day one, and then 2,2 mg/kg daily.

11. Use according to anyone of the previous claims 3 to 10, wherein melarsoprol is administered according to intravenous injections, preferentially, three per day.

12. Solution containing melarsoprol at concentration of 36 mg/ml for treating multiple myeloma.

13. Use according to anyone of the previous claims 3 to 11 wherein melarsoprol is in form of a injectable solution.

14. Use according to claim 13 wherein the concentration of melarsoprol in the injectable solution is 36mg/ml.

15. Process of preparation of a medicament for treating B-cell lymphoproliferative disorders characterized in that melarsoprol is the main active ingredient of the said medicament.
